# EUROPEAN PATENT APPLICATION

(11) **EP 1 382 681 A1**
(43) Date of publication of application: **21.01.2004**
(21) Application number: 02090251.6
(22) Date of filing: 15.07.2002
(51) Int. Cl.: C12N 15/62, C07K 5/08, C07K 14/00

(54) **A modified CBD/RGD recombinant attachment factor for improving cell-attachment efficiency and the manufacturing method thereof**

(71) Applicant: Bio999 Inc., Taichung, Taiwan 402 (CN)
(72) Inventor: Lin, Chia-Hui, Taichung, Taiwan 408 (TW)
(74) Representative: Hengelhaupt, Jürgen, Dipl.-Ing.

(57) **Abstract**

A modified CBD/RGD recombinant attachment factor for improving cell attachment efficiency and the manufacturing method thereof, wherein the Arg-Gly-Asp (RGD) amino acid sequence is grafted on the C-terminal having a cellulose binding domain (CBD), thus the CBD-RGD polypeptide is capable of promoting the cell to attach onto the cellulose culturing plate; and as an RGD sequence is added, it can be accommodated in a stable annular structure built up by disulfide bonds, and is grafted on the N-terminal of said cellulose binding domain (CBD); thus, a CBD/RGD recombinant attachment factor for improving the promotion capability of cell-attachment is composed, and therefore the promotion capability of cell-attachment can be enhanced conspicuously.

## Description

### Background of the Invention

### 1. Field of the Invention

The present invention relates to a method for improving cell-attachment efficiency, especially to a modified CBD/RGD recombinant attachment factor for improving cell-attachment efficiency.

### 2. Description of the Related Industries

Most of the animal cells for producing protein are capable of dependence of attachment that is utilized for attaching on the extracellular glycoproteins such that the fibronectin so as to benefit the growth, spreading and differentiation. Serum is an abundant resource of the glycoproteins (please refer to: Barnes, D.W., Silnutzer, J. 1983 J. Biol. Chem. 258: 12548-12552), but it is rather expensive and difficult to be isolated, and so there is a restriction on mass cell-culturing (referring to: Arathoon, W.R., Birch, J.R. 1986. Science 232:1390-1395). Only if no serum is added while culturing, the cost would be reduced, and the dangers of being infected with microbes or viruses from the animal serum would also be prevented. As the cells is high-density cultured in the culturing liquid without the serum for several months, much more quantities of proteins would be produced (referring to: Vournakis, J.N., Runstadler, P.W. 1989 Bio/ Technology 7:143-145). Moreover, since the serum protein is not included in the culturing liquid, it is much easier to purify the target protein, and the cost thereof is lowered. Further, if the contents of the culturing liquid were adjusted adequately, the whole manufacturing process would become ideal.

The culturing of the cells with dependency of attachment in the culturing liquid without serum is in need of some additional attachment factors (referring to: Clement, B., Segui-Real, B., Savagner, P., Kleinnam, H.K., Yamada, Y. 1990. J. Cell Biol. 110: 185-192). It is particularly important for mass-culturing the cells on the microcarriers since the initial conditions for attaching the cells are relatively severe (referring to: Steele, J.G, Johnson, G, Norris, W.D., Underwood, P.A. 1991 Biomaterials 12:531-539). In 1967, the microcarrier is first disclosed by Wezel (referring to: Wezel, A. L. V 1967. Nature. 216:64-65), which is provided with larger unit growth area by means of the electrified beads; thus the wall-attached cells are enabled to adsorb on the beads, and are mass-cultured. Furthermore, it is designed to be coordinated with various bioreactors for mass-producing cells and the cell-products (referring to: Chen, Z., Y Chen and Y. Shi. 1992, Can. J. Microbiol. 38: 222-225. Pierre, J., J. Lapierre, C. Daniel, R. Dugre and P. Trepanier. 1989. J. Virol. Methods. 25:63-70. Hu, W. S. 1985. Biotech. Bioeng. 27:585-595). Henceforth, the immobilized cell culture technology is promptly analyzed and developed, and the development of the immobilized cell materials is one of the focuses of the immobilized cell culture technology. The immobilized cell materials that are broadly applied include dextran with positive charge (DEAE, diethylaminoethyl), collagens, and also hollow fiber, ceramic matrices, etc. Most of the immobilized cell materials are commercialized, and they are effective but expensive. In the past, the natural cellulose once were used as the material for culturing the immobilized cell by the researchers; the natural cellulose has the advantages of providing large surface area for growth, low cost, availability, and corresponding with the environmental protection; nevertheless, the attachment growth efficiency of the animal cells grown on the cellulose materials is not desirable (referring to: Wierzba, A., U. Reichl, R. F. B. Turnner, R.A.J. Warren and D. G Kilburn. 1995. Biotech. Bioeng. 47:147-154; Wierzba, A., U. Reichl, R. F. B. Turnner, R. A. J. Warren and D. G Kilburn. 1995. Biotech. Bioeng. 47:185-193). In addition, the extracellular matrix glycoproteins can be added into the growth culturing liquid additionally, but high cost for purification and pollution are also happened; therefore, it is seriously required for developing the recombinant attachment factors.

### 3. Description of the Prior Art

Recently, the oncology researchers have put emphasis on cell-attachment, spreading, differentiation and migration as the cells are in vivo; most of the normal cells are fixed on the extracellular matrix as the cells are in vivo (referring to: Freshney, R. I. 1986. Animal Cell Culture 2^{nd} ed. Oxford University Press, New York). The extracellular matrix is formed of biosynthesis products being transferred outside the cell membrane and then being piled up, and it is a way that the absorption of nutrition and pathogen and the excretion of metabolism must pass by. The extracellular matrix includes some typical non-collagen attachment proteins having multiple domains; each domain is provided with a special combination sequence for grafting matrix macromolecule on cell surface receptor. The proteins are capable of recombining the matrix and supporting the cells being attached onto the matrix. Wherein, a well-known one is the glycoprotein macromolecule of the fibronectin protein that can be found inside the cells of all vertebrates, and it is considered as being capable of cell-attachment, differentiation and migration (referring to: Hynes, R. O. 1986. Fibronectins. Sci. Am. 254:42-51; Freshney, R. I. 1994. Culture of Animal Cell. 3^{rd} ed. Wiley Liss, New York). The glycoprotein macromolecule of the fibronectin protein is composed of two subunits, and the two subunits are connected with each other by a disulfide bond on the C-terminals of the two subunits. Each subunit is folded up and thus a series of domains is formed that are separated by a bendable polypeptide chain, and the domains include: collagen-binding, fiber-binding, heparin-binding, and cell-binding, etc. (referring to: Hynes, R. O. 1990. Fibronectin. 1^{st} ed. Springer-Verlag, New York). After the fibronectin is proceeded with the low-concentrated protein hydrolytic enzyme, one of the domains is enabled to be bound to the collagen, and other domains are capable of binding to the heparin and other special receptors located at the surfaces of various cells. It is demonstrated by the scientists that there are three special amino acid sequences - arginine-glycine-aspartate (RGD) - on the domain for promoting cell-attachment, and it is a minimum functional unit for promoting cell-attachment of the fibronectin (referring to: Ruoslahti, E. and M. D. Pierschbacher. 1987. Science. 238: 491-496). If the amino acid is chelated to the surface of the cell culturing materials, it is beneficial to bind the cell to the solid materials. The RGD sequence appears not only on fibronectin but also on other extracellular matrix glycoproteins. The RGD sequence takes effect depending on the RGD sequence is located at a position in the whole protein structure that easily to be affected by the outwards (referring to: Hautanen, A., J. Gailit, D. M. Mann and E. Ruoslahti. 1989. J. Biol. Chem. 264: 1437-1442). For example, in the fibronectin, the RGD sequence for promoting cell-attachment is located at the β-turn, and is exposed outmost on the whole structure (referring to: D'Souza, S. E., M. H. Ginsberg and E. F. Plow. 1991. TIBS. 16: 246-250). In addition, it seems that the activity of the RGD sequence is affected by the adjacent several amino acid molecules or the further ones, and the actual reason is still unknown (referring to: Ruoslahti, E. and Y. Yamaguchi. 1991. Cell. 64:867-869: Tranqui, L., A. Andrieux, G Hudry, J. Clergeon, J. Rychewaert, S. soyze, A. Chapel, M. H. Ginsberg, E. F. Plow and G Marguerie. 1989. J. Cell Biol. 108:2519-2527). By means of the cellular matrix being bound with the receptors of the cell surface, a series of biochemical reactions is triggered; thus, the physiology and morphology of the cell are changed and the whole structure of the cell is influenced. If the cells are grown in the matrix formed of fibronectin, the stress fibers in the cells would be spread and adjusted by the cell, and then arranged together with the fibronectin filaments outside the cells; such interaction is controlled by the integrin family. As in vivo, the RGD sequences on the fibronectin can by recognized by the integrin, so as to rearrange the internal structure of the cell, and enable the cells to attach onto the extracellular matrix steadily; the integrin is a very important receptor protein on the cell surface. The integrin is formed of two transmembrance-glycoprotein-subunits α and β, both subunits can be bound with the matrix protein. The recognition domain of the RGD sequence is located at the subunit β; the subunit α is bigger, and it is provided with a heavy chain and a light chain, located at the heavy chain outside the cell membrane, and can be bound with divalent positive ions (D'Souza, S. E., M. H. Ginsberg and E. F. Plow. 1991. TIBS. 16: 246-250).

If the RGD sequence polypeptide, which is capable of cell-attachment, is bound with the protein, which is capable of cellulose binding, it is used as an additive for cell culturing so as to enable the cell to attach onto the cellulose. Therefore, not only the cost of culturing the animal cells and purifying the proteins is lowered, but also the possible contamination of protein preparation is prevented. It deserves to be advanced that it is a cell culturing material for cellulose immobilization.

### 4. Description of the Latest Art

In the past, the resembling combinations have been in progress by the scientists: firstly in 1995 by Wierzba, A., the CBD gene in the endoglucanase A (Cen A) of the cellulomonas fimi, a bacteria, is grafted onto a synthetic RGD sequence gene, and an active protein of 17 kDa is presented (referring to: Wierzba, A., U. Reichl, R. F. B. Turnner, R. A. J. Warren and D. G Kilburn. 1995. Biotech. Bioeng. 47: 147-154). The Cen A from the bacteria is also a cellulose digestive enzyme; it has a catalytic domain and a cellulose binding domain, wherein the two domains are connected by a linker having abundant proline and theronine. The catalytic domain of the Cen A is located at the C-terminal, and the CBD is located at the N-terminal, so the RGD sequence is grafted directly to the N-terminals of the natural CBD and PT linker in the test by Wierzba, A. et al. (referring to: Din, N., I. J. Forsythe, L. D. Burtunick, N. R. Gilkes, R. C. Miller, A. J. Warren and D. G Kilburn. 1994. Mol. Microbiol. 11: 747-755).

Besides, by Y. Z. Chen (referring to the Master Essay by Y Z. Chen from Taiwan National Chung-Hsing University, 1997), the RGD sequence is grafted on the CBH I gene of the bacteria of Trichoderma koningii, and the basic composition thereof is very similar to the Cen A gene but the catalytic domain of the CBH I gene is located at the N-terminal, CBD is located at the C-terminal, and it is composed of 36 amino acids (referring to: Wey, T. T., T. H. Hseu and L. Huang. 1994. Cur. Microbiol. 28:31-39). It is disclosed by Chen that a new PT linker is grafted on the C-terminal of CBD, and then to the RGD sequence. It is improved according to the present invention that an RGD sequence is added and existed in a stable annular structure built up by a disulfide bonds, and it is grafted to the N-terminal of the cellulose binding domain (CBD). It's demonstrated that the of cell-attachment-promoting efficiency by the RGD with annular structure is better than that of the RGD with linear structure, such as the RGD sequence of venin protein (referring to: D'Souza, S. E., M. H. Ginsberg and E. F. Plow. 1991. TIBS. 16: 246-250). The RGD sequence can be found in various venin protein, such as rhodotomin (Rho), trigramin; it is capable of recognizing and grafting the integrin of the platelets, and then restraining the platelets from coagulation (referring to: Huang, T. F., J. H. Holt, H. Lukasiewiez and S. Niewiarowai. 1987. J. Biol. Chem. 262:16157-16163). Such proteins usually contain 8-14 cysteine fragments, thus many disulfide bondss are formed, and the RGD sequence can exist in the stable annular structure built up by the disulfide bonds (referring to: Knudsen, K. A., G. P. Tuszynski, T. F. Huang and S. Niewiarowski. 1988. Exp. Cell. Research. 179:42-49). It is tested and verified that the promotion capability of cell-attachment is enhanced conspicuously according to the present invention.

### Summary of the Invention

Accordingly, it is not accomplished that the expectation of the capability for promoting cell-attachment of the CBD/RGD attachment factor according to the prior art, It is an object of the present invention to provide a CBD/RGD recombinant attachment factor with improved cell-attachment efficiency, which enables the Arg-Gly-Asp (RGD) amino acid sequence for controlling the cell-attachment to be grafted on a C-terminal with the cellulose binding domain (CBD). Thus, by the CBD-RGD polypeptide, the cell can be enabled to attach onto the cellulose-culturing container, an RGD sequence is additionally added; it can be accommodated in a stable annular structure built by disulfide bonds, and is grafted on the N-terminal with the CBD, so as to form a CBD/RGB recombinant attachment factor for enhancing the promotion capability of cell-attachment conspicuously.

The present invention will be better understood and its numerous objects and advantages will become apparent to those skilled in the art by referencing to the following drawings.

### Brief Description of the Drawings

Fig. 1 is a perspective view showing the composition of the recombinant attachment factor according to the present invention.
Fig. 2 is an image taken by a digital camera showing the analysis of the protein that is expressed and purified on the SDS-PAGE.
Figs. 3 and 4 are comparative views showing the recombinant attachment factors of the present invention and the prior art.
Fig. 5 shows the cell-attachment situations taken by a 100X digital microcamera.

### Detailed Description of the Preferred Embodiment

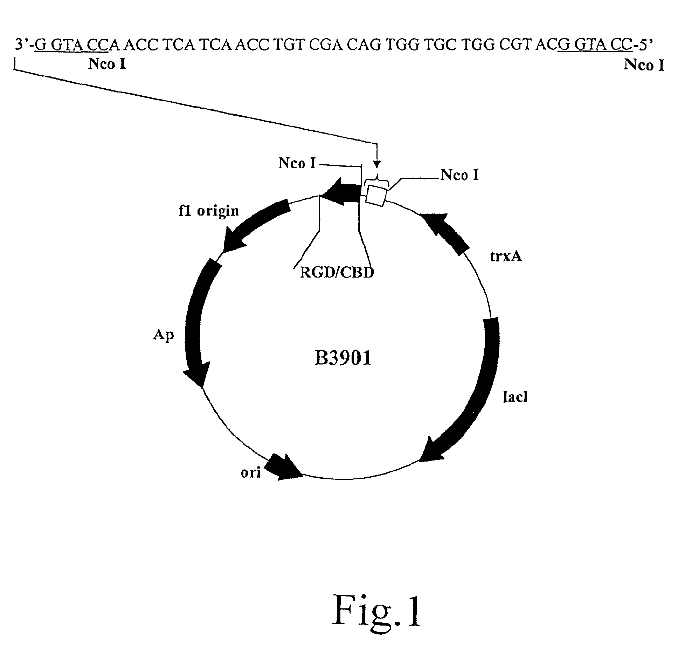

By means of the standard skill (disclosed by Joseph Sambrook and David W. Russell, Molecular Cloning), E. coli strain named B3901 is cloned. A synthetic primer of: 5'CATGCCATGG CATGCGGTCG TGGTGACAGC TGTCCAA 3' 3'CCACTGTCGA CAGGTTGATG AGGTTGGTAC CGTAC 5' is enlarged by the poly chain reactor, and is cut by a restriction enzyme of Nco I, then it is mixed with a standard solution and connected with the RGD sequence such as the one composed by Chen, and it is grafted on the C-terminal of CBD by a PT linker. The grafted primer is put in the expression plasmid model of pET32a (referring to: Lavallie, E. R., E. A. DiBlasio, S. Kovacic, K. L. Grant, P. F. Schendel and J. M. McCoy. 1993. Bio/Technology 11:187-193; Fig. 1), and it is transformed directly to be BL21 (DE3) E. coli by the steps of standard CaCl₂, then it is propagated and cultured in a flat plate, and finally, the plasmid DNA in a single colony is selected, and the strain with recombinant plasmid can be sifted out by means of the restriction enzyme and the poly enzyme reaction.

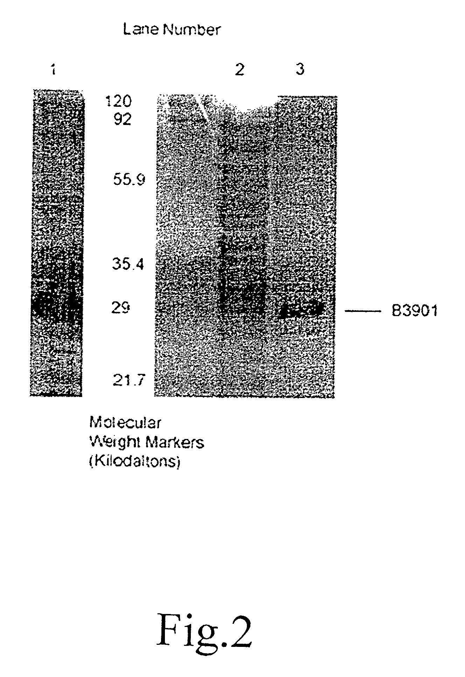

The constructed B3901 E. coli strain is cultured in the TB medium including 0.05 µg/ml ampicillin until O.D. 600 = 0.7∼1, and 1mM IPTG is added for bringing out the expression thereof A bacteria sample of 7000g is gathered after it is centrifugalized in 4 °C for 10 minutes, and it is suspended in a buffer solution of Tris-HC1 (pH 7.4), wherein 0.1-mM PMSF and 1-mM EDTA is added in order to restrain the protease activity of the hydrolysis target protein before being purified. The cell wall of the bacteria is broken by a French Press^{TM} with a 16-kpsi stress, then 10000g of it is centrifugalized in 4 °C for 15 minutes. Since protein expressed by the pET32a expression plasmid includes a zone of 6 histidine, the above-mentioned liquid (after the SDS-PAGE analysis, it is found that a protein is expressed considerably where the molecular weight is about 26 kDa, as shown in Fig. 2, lane 1) is filtered by a 0.45-µm membrane, and purified by passing directly through a Ni-chelated affinity column. Firstly, the column is rinsed by 20-mM Imidazole with a quantity of 5 to 10 times of the column, so as to rinse away the redundant, non-bound proteins (as shown in Fig. 2, lane 2). Secondly, the column is rinsed by 50∼100-mM Imidazole with a quantity of 5 to 10 times of the column, so as to rinse away the non-specific-bound proteins and the weakly-bound proteins. Finally, the target protein is taken away from the specific-bound affinity column by 250-mM Imidazole and collected. 0.1% coomassie blue is added into the collected sample, then heated for 5 minutes, and proceeded with the electrophoresis analysis on the SDS-PAGE. For producing attachment factor, the 26-kDa protein signals (as shown in Fig.2, Lane 3) would appear, then the CBD/RGD recombination attachment factor according to the present invention is accomplished.

### Example 1

Here, the comparison of the attachment efficiency between the original CBD/RGD and the B3901 of the present invention is listed below:

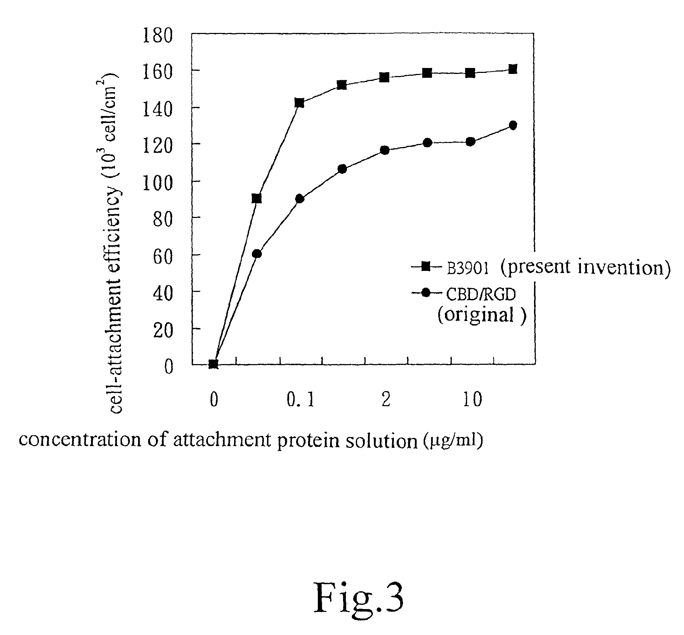

The attachment proteins are diluted as solutions having various values of concentration: 0.01 µg/ml, 0.1 µg/ml, 1 µg/ml, 2 µg/ml, 5 µg/ml, 10 µg/ml, 100 µg/ml (as shown in Fig.3). They are dropped into the 24-hole culturing plate that is treated with cellulose acetate respectively, and kept in a room temperature for about 2 or 4 hours for proceeding reactions so as to remove the non-bound proteins, then 2-µg/ml DMEM cell culturing liquid of BSA is added into each hole of the plate and kept in 37 °C. After the Maden Darby Bovine Kidiney cell is removed from the plate by trypsin, it is rinsed by a SMEM culturing liquid having 0.01% soybean trypsine inhibitor in order to inhibit the operation of the trypsin. Afterwards, it is rinsed twice by DMEM culturing liquid (FBS-free), and 1.6×10⁵ cells are taken out and cultured in each hole having different concentration of attachment protein for 1.5 hour, then the number of the attachment cells is counted, and consequently, the best concentration of the CBD/RGD attachment protein is 2∼5 µg/ml, and that of B3901 is 0.1∼1 µg/ml (Mean ± SEM of six individual tests). By comparison, B3901 can achieve good attachment efficiency with low concentration.

### Example 2

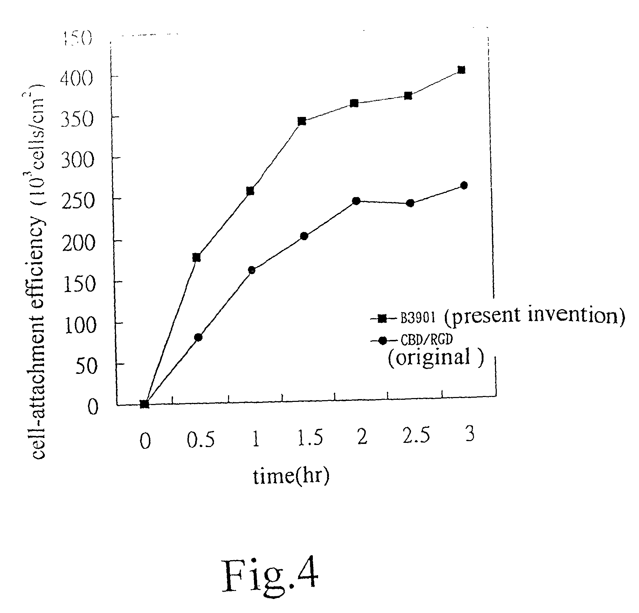

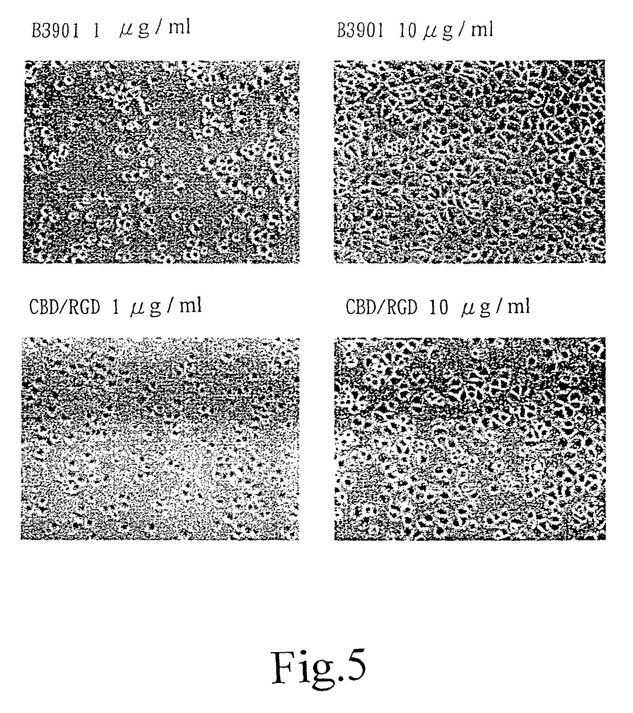

According to the test result above, by using a familiar method, 4x10⁵ cells are taken out and the protein concentration is maintained, such as the protein concentration of CBD/RGD is 2∼5 µg/ml and that of B3901 is 0. 1∼1 µg/ml, then a kinetics statistics of cell-attachment efficiency according to various points of time is carried out (as shown in Fig. 4). Consequently, the tests of B3901 result in up to 100% cells are attached onto the culturing plate just at the point of 1.5 hour, and the tests of CBD/RGD proteins result in only 50% cells are attached onto the culturing plate at the same moment (Mean ± SEM of 6 individual tests). It shows that the cell-attachment-promoting efficiency of the modified proteins according to the present invention is better than that of the original CBD/RGD, conspicuously. Wherever the MDBK cell is attached to the CBD/RGD attachment protein or to the modified B3901 attachment protein of the present invention, both the morphology and size are similar, but obviously, it can be seen in Fig. 5 that the modified cell-attachment efficiency of the present invention is much better

### Benefit of the Present Invention

By developing of the attachment protein, not only the cost of purifying protein or manufacturing bacterin can be lowered, but also the contamination by microorganism and virus in animal serum can be prevented as well, thus it benefits the biotechnology industries and so that it is a product that is worth to be developed admittedly. In accordance with cell-attachment-promoting efficiency of the original CBD/RGD recombinant protein attachment factor (by Y. Z. Chen, Master Essay of Taiwan National Chung-Hsing University, 1977) is out of expectation, the present invention is generated by modifying and simulating the natural physiology and phenomenon, and it enables the RGD sequence to exist in a stable annular structure built up by a disulfide bond. It is demonstrated that the attachment efficiency of the modified cell-attachment-promoting factor of the present invention is enhanced, and the concentration of the solution for accomplishing the maximum attachment efficiency is comparatively low, thus it is highly beneficial to the industries.

Although the present invention has been described using specified embodiment, the examples are meant to be illustrative and not restrictive. It is clear that many other variations would be possible without departing from the basic approach, demonstrated in the present invention.

## Claims

1. A modified CBD/RGD recombinant attachment factor for improving cell attachment efficiency, wherein the Arg-Gly-Asp (RGD) amino acid sequence is grafted on the C-terminal having a cellulose binding domain (CBD), thus the CBD-RGD polypeptide is capable of promoting the cell to attach onto the cellulose culturing plate; and as an RGD sequence is added, it can be accommodated in a stable annular structure built up by disulfide bonds, and is grafted on the N-terminal of said cellulose binding domain (CBD); thus, a CBD/RGD recombinant attachment factor for improving the promotion capability of cell-attachment is composed.

2. The CBD/RGD recombinant attachment factor as claimed in claim 1, wherein the RGD sequence is accommodated in a stable annular structure built up by disulfide bonds of cysteine.

3. A method for manufacturing the modified CBD/RGD recombinant attachment factor for improving cell attachment efficiency, which includes the steps as below:
constructing a colon bacillus E. coli. Firstly;
enlarging the synthetic primer of 5'CATGCCATGG CATGCGGTCG TGGTGACAGC TGTCCAA 3' 3’CCACTGTCGA CAGGTTGATG AGGTTGGTAC CGTAC 5’
with a poly chain reactor, and cutting it with a restriction enzyme of Nco I, then by mixing in the standard solution, it is grafted on the RGD sequence, which is disclosed by Z., Y. Chen, with a PT linker connecting to the C-terminal of the CBD;
Putting the grafted primer into a model of pET32a expression plasmid, then by applying the standard CaCl₂ process, transforming it into the BL21(DE3) E. coli by the connecting solution directly, and propagating, culturing it on a medium;
Getting the plasmid DNA of a single colony, and by using the restriction enzyme coordinating with the poly chain reaction, sifting the strain having recombinant plasmid out;
Culturing the constructed E. coli strain to be O.D. 600=0.7∼1 by the TB medium having 0.05-µg/ml Ampicillin;
Adding 1-mM IPTG for carrying the expression out, centrifuging 7000g of it in 4 °C for 10 minutes, and collecting the bacteria sample;
Suspending the bacteria in a 20-mM Tris-HCl buffer solution (pH 7.4) having 0.1-mM PMSF and 1-mM EDTA; the additional EDTA and PMSF are capable of restraining the protease activity of the hydrolysis target protein before it is purified;
Breaking the cell wall of the bacteria by a press, and centrifuging 10000g of it in 4 °C for 15 minutes;
Filtering it by a 0.45-µm membrane, then purifying it directly by passing through a Ni-chelate affinity column;
Rinsing and balancing the column by a 20-mM Imidazole solution with a quantity of 5∼1 times of the column, so as to remove the redundant, non-bound protein;
Rinsing the column by a 50∼100-mM Imidazole solution with a quantity of 5∼10 times of the column, so as to flush the non-specific-bound protein and the weakly-grafted protein out of the column; and
Collecting the target protein out of the specific-bound affinity column by flushing it out.
